# EUROPEAN PATENT APPLICATION

(11) **EP 4 151 198 A1**
(43) Date of publication of application: **22.03.2023**
(21) Application number: 21804042.6
(22) Date of filing: 14.05.2021

(54) **GOLD NANOPARTICLE-CONTAINING MEDICINE**

(30) Priority: 15.05.2020 JP 2020086250; 16.02.2021 JP 2021022612
(71) Applicant: OSAKA UNIVERSITY, Suita-shi Osaka 565-0871 (JP)
(72) Inventor: KATO Hiroki, Suita-shi, Osaka 565-0871 (JP); FUKASE Koichi, Suita-shi, Osaka 565-0871 (JP); KABAYAMA Kazuya, Suita-shi, Osaka 565-0871 (JP); SHIMOYAMA Atsushi, Suita-shi, Osaka 565-0871 (JP); KADONAGA Yuichiro, Suita-shi, Osaka 565-0871 (JP); TOYOSHIMA Atsushi, Suita-shi, Osaka 565-0871 (JP); SHINOHARA Atsushi, Suita-shi, Osaka 565-0871 (JP); KANEDA Yasufumi, Suita-shi, Osaka 565-0871 (JP); NISHIKAWA Tomoyuki, Suita-shi, Osaka 565-0871 (JP)
(74) Representative: Nederlandsch Octrooibureau
(86) International application number: PCT/JP2021/018485
(87) International publication number: WO 2021/230369

(57) **Abstract**

The present invention relates to a gold nanoparticle-containing medicine, and a treatment of a proliferative disease using the medicine. The present invention also relates to a gold nanoparticle-containing medicine that is bound to an alpha radioactive nucleus, and a treatment of a proliferative disease using the medicine.

## Description

### Technical Field

The present invention relates to a gold nanoparticle-containing medicine, and a treatment of a proliferative disease using the medicine. The present invention also relates to a gold nanoparticle-containing medicine that is bound to an alpha radioactive nucleus, and a treatment of a proliferative disease using the medicine.

### Background Art

For the treatment of malignant tumors including brain tumors, surgery, chemotherapy by systemic administration of an antitumor drug, and radiation therapy by external gamma irradiation are applied. These therapies alone are less effective in many cases, and it is important to perform as many treatment methods as possible in a multidisciplinary manner, but each of the treatment methods has a problem. Treatment with surgery is highly invasive, and chemotherapy by systemic administration has very strong systemic side effects. Radiation therapy by gamma irradiation has a radiation exposure problem due to a relatively long range. In recent years, treatment methods have been developed in which a conventional antitumor drug is administered to a malignant tumor, but effects of these methods have not yet been established.

It is known that cytotoxicity of radiation varies depending on its radiation quality. Alpha rays have a much higher linear energy transfer (LET) over gamma rays and beta rays heretofore used for treatment (Non Patent Literature 1), and have a particularly high antitumor action. In addition, the range of an alpha ray is very short, so that only a narrow area is affected. Therefore, it is required that an alpha radioactive nucleus be diffused in a lesion and prevented from being distributed to normal tissues. Heretofore, as an attempt to use an alpha radioactive nucleus for treatment of a proliferative disease such as a tumor, it has been proposed to add a targeting molecule that specifically binds to target tumor cells or has affinity for the target tumor cells, etc. (Non Patent Literatures 1 and 2). However, in such methods, it is necessary to select an optimal targeting molecule for each tumor, and therefore a more versatile technique for applying an alpha radioactive nucleus to the treatment of a proliferative disease is required.

The present inventors have previously demonstrated for the first time that AuNP(At)-PEG with alpha radioactive nucleus astatine 211 (At-211) and polyethylene glycol (PEG) bound to gold nanoparticles (AuNP) is useful for direct administration to glioma. It has been found that since the range of an alpha ray of AuNP(At)-PEG is very short, the diffusibility of particles in a tumor tissue is important, and for this reason, it is most important to adjust the particle size. However, heretofore, optimization of the particle size has not been performed, and the effect of administration of the particles to a living body has not been proved.

### Citation List

### Non Patent Literature

Non Patent Literature 1: Royal Society of Chemistry, 2017, Vol. 4, pp. 41024-41032
Non Patent Literature 2: Nanomaterials, 2019, Vol. 9, doi.org/10.3390/nano9040632, pp. 1-15

### Summary of Invention

### Technical Problem

An object of the present invention is to provide a medicine in which the size of gold nanoparticles is optimized to regulate diffusion characteristics in tumor cells and the like, so that an excellent treatment effect is exhibited.

### Solution to Problem

The present inventors have paid attention to gold nanoparticles (AuNP) having no toxicity, added polyethylene glycol (PEG) for securing diffusibility in a tissue fluid, and conducted intensive studies on the particle size in order to achieve high diffusion in a tissue and non-systemic diffusion. Resultantly, for the first time, a glioma treatment drug for topical administration has been provided which ensures that use of a selective targeting molecule for a specific cell is not required, an alpha radioactive nucleus is locally retained only in a tumor for a long time, a strong antitumor action is applied to nearby malignant cells, the drug is not diffused to organs throughout the body, and there are no side effects.

In an aspect of the present invention, a treatment effect is obtained by directly administering an alpha radioactive nucleus to a proliferative disease using the gold nanoparticles (AuNP).

In an aspect, the present invention provides a medicine for treating a proliferative disease, which contains gold nanoparticles having a particle size of 0.5 to 110 nanometers and bound to At-211, and is topically administered.

In an aspect, the surface of the gold nanoparticle may be modified with a molecule selected from polyethylene glycol, polyether, polyol, polyethyleneimine, silica gel, a peptide, an antibody, a protein, a lipid, a complex lipid, a sugar chain, a complex carbohydrate, terpene, terpenoid, and a virus-like particle.

In an aspect, the surface modification involves a molecule that is not bound to a targeting molecule for a specific cell, and in still another aspect, the surface modification does not involve a targeting molecule for a specific cell.

In an aspect, the molecule that is not bound to a targeting molecule for a specific cell may be polyethylene glycol having an average molecular weight of 2,000 to 20,000.

In an aspect, the particle size of the gold nanoparticle may be 0.5 to 13 nm.

In an aspect, the medicine of the present invention can be administered by topical administration selected from the group consisting of injection into a lesion, super-selective administration into an artery feeding the lesion, and intracavity application.

In an aspect, the proliferative disease is a malignant tumor, and may be a solid cancer.

In an aspect, the solid cancer may be selected from a brain tumor, endocrine tumors, prostate cancer, head and neck cancer, oral cancer, breast cancer, gynaecological cancer, skin cancer, pancreas cancer, and digestive organ cancer.

In another aspect, the present invention provides gold nanoparticles which have a particle size of 0.5 to 110 nanometers, bind to At-211 and include a surface modification with polyethylene glycol that is not bound to a targeting molecule for a specific cell.

In an aspect, the gold nanoparticles of the present invention may further include a surface modification with a targeting molecule for a specific cell.

In another aspect, the present invention provides use of gold nanoparticles having a particle size of 0.5 to 110 nanometers for manufacturing a medicine for treating a proliferative disease, which is topically administered.

In an aspect, the gold nanoparticles are bonded to At-211.

In an aspect, the surface of the gold nanoparticle may be modified with a molecule selected from polyethylene glycol, polyether, polyol, polyethyleneimine, silica gel, a peptide, an antibody, a protein, a lipid, a complex lipid, a sugar chain, a complex carbohydrate, terpene, terpenoid, and a virus-like particle.

In an aspect, the surface of the gold nanoparticle may be modified with polyethylene glycol having a molecular weight of 2,000 or more.

In another aspect, the present invention provides a method for treating a proliferative disease by topically administering gold nanoparticles having a particle size of 0.5 to 110 nanometers and bound to At-211.

In an aspect, the surface of the gold nanoparticle may be modified with a molecule selected from polyethylene glycol, polyether, polyol, polyethyleneimine, silica gel, a peptide, an antibody, a protein, a lipid, a complex lipid, a sugar chain, a complex carbohydrate, terpene, terpenoid, and a virus-like particle.

In an aspect, the surface of the gold nanoparticle may be modified with polyethylene glycol having a molecular weight of 2,000 or more.

In an aspect, the topical administration can be selected from the group consisting of injection into a lesion, super-selective administration into an artery feeding the lesion, and intracavity application.

In an aspect, the proliferative disease is a malignant tumor, and may be a solid cancer.

In an aspect, the solid cancer is selected from a brain tumor, endocrine tumors, prostate cancer, head and neck cancer, oral cancer, breast cancer, gynaecological cancer, skin cancer, pancreas cancer, and digestive organ cancer.

In another aspect, the present invention provides a method for selecting a particle size of At-211-bound gold nanoparticles which is optimum for treating a proliferative disease by topical administration, the method comprising the steps of:
(1) providing At-211-bound gold nanoparticles having different particle sizes ranging from 0.5 to 110 nanometers;
(2) administering the At-211-bound gold nanoparticles having respective particle sizes into a proliferative disease tissue in vivo;
(3) confirming an alpha ray distribution in the proliferative disease tissue subjected to the administration, and a systemic alpha ray distribution; and
(4) selecting a particle size on the basis of the alpha ray distribution in the proliferative disease tissue, and the systemic alpha ray distribution.

In an aspect, the method may comprise, in addition to the step (4), the step (5) of evaluating a change in body weight of an animal subjected to the administration, and/or an inhibitory effect on proliferation of the proliferative diseased tissue.

In an aspect, the topical administration into the proliferative disease tissue can be selected from the group consisting of injection to the central part in the tissue, super-selective administration into an artery feeding the lesion, and application of the drug into a cavity where the tissue is present.

In an aspect, the in vivo proliferative disease tissue may be a heterogeneous-proliferative disease tissue transplanted into a subject.

In an aspect, the surface of the gold nanoparticle may be modified with a molecule selected from hydrocarbon-based polymers such as polyethylene glycol, polyether and polyol, polyethyleneimine, silica gel, a peptide, an antibody, a protein, a lipid, a complex lipid, a sugar chain, a complex carbohydrate, terpene, terpenoid, and a virus-like particle. In an aspect, the surface of the gold nanoparticle may be modified with polyethylene glycol having a molecular weight of 2,000 or more.

In an aspect, the proliferative disease is a malignant tumor, and may be a solid cancer. In an aspect, the solid cancer is selected from a brain tumor, endocrine tumors, prostate cancer, head and neck cancer, oral cancer, breast cancer, gynaecological cancer, skin cancer, pancreas cancer, and digestive organ cancer.

In another aspect, the present invention provides a method for manufacturing At-211-bound gold nanoparticles having an optimum particle size for treating a proliferative disease by topical administration, the method comprising the steps of:
(1) providing At-211-bound gold nanoparticles having different particle sizes ranging from 0.5 to 110 nanometers;
(2) administering the At-211-bound gold nanoparticles having respective particle sizes into a proliferative disease tissue in vivo;
(3) confirming an alpha ray distribution in the proliferative disease tissue subjected to the administration, and a systemic alpha ray distribution; and
(4) selecting a particle size on the basis of the alpha ray distribution in the proliferative disease tissue, and the systemic alpha ray distribution.

In an aspect, the method may comprise, in addition to the step (4), the step (5) of evaluating a change in body weight of an animal subjected to the administration, and/or an inhibitory effect on proliferation of the proliferative diseased tissue.

In an aspect, the topical administration into the proliferative disease tissue can be selected from the group consisting of injection to the central part in the tissue, super-selective administration into an artery feeding the lesion, and application of the drug into a cavity where the tissue is present.

In an aspect, the in vivo proliferative disease tissue may be a heterogeneous-proliferative disease tissue transplanted into a subject.

In an aspect, the surface of the gold nanoparticle may be modified with a molecule selected from hydrocarbon-based polymers such as polyethylene glycol, polyether and polyol, polyethyleneimine, silica gel, a peptide, an antibody, a protein, a lipid, a complex lipid, a sugar chain, a complex carbohydrate, terpene, terpenoid, and a virus-like particle. In an aspect, the surface of the gold nanoparticle may be modified with polyethylene glycol having a molecular weight of 2,000 or more.

In an aspect, the proliferative disease is a malignant tumor, and may be a solid cancer. In an aspect, the solid cancer is selected from a brain tumor, endocrine tumors, prostate cancer, head and neck cancer, oral cancer, breast cancer, gynaecological cancer, skin cancer, pancreas cancer, and digestive organ cancer.

### Advantageous Effects of Invention

The alpha nucleus-containing nanoparticles of the present invention have excellent diffusibility in a tumor tissue and low systemic diffusibility, and is less injurious to other organs while effectively suppressing proliferation of a proliferative disease. In addition, it is possible to provide a medicine which can be repeatedly administered to a proliferative disease tissue at an extremely high dose because of little necessity to give consideration to exposure, and exhibits a high inhibitory action on proliferation of a proliferative disease tissue.

### Brief Description of Drawings

Fig. 1 illustrates the results of scintigraphic analysis in administration of 120 nm AuNP(At)PEG to a subcutaneously transplanted glioma model rat.
Fig. 2 illustrates the results of scintigraphic analysis in administration of 30 nm AuNP(At)PEG to a subcutaneously transplanted glioma model rat.
Fig. 3 illustrates the results of scintigraphic analysis in administration of AuNP(I-123)PEG to a subcutaneously transplanted glioma model rat.
Fig. 4 illustrates the results of autoradiography in administration of 120 nm AuNP(At)PEG or 30 nm AuNP(At)PEG to a subcutaneously transplanted glioma model rat.
Fig. 5 illustrates the results of autoradiography in administration of 30 nm non-PEG-modified AuNP(At) to a subcutaneously transplanted glioma model rat.
Fig. 6 illustrates a change in tumor size in administration of 5 nm, 13 nm, 30 nm or 120 nm AuNP(At)PEG to a subcutaneously transplanted glioma model rat.
Fig. 7 illustrates a change in body weight in administration of 5 nm, 13 nm, 30 nm or 120 nm AuNP(At)PEG to a subcutaneously transplanted glioma model rat.
Fig. 8 illustrates a mass of a tumor tissue extracted on day 42 after administration of 5 nm, 13 nm, 30 nm or 120 nm AuNP(At)PEG to a subcutaneously transplanted glioma model rat.
Fig. 9 illustrates the results of scintigraphic analysis performed 4, 19 and 42 hours after injection of 5 nm AuNP(At)PEG particles to a subcutaneously transplanted glioma model rat.
Fig. 10 illustrates a change in tumor size in administration of AuNP(At)PEG or AuNPPEG (non-labeled) to a subcutaneously transplanted kidney cancer model mouse.
Fig. 11 illustrates a change in body weight in administration of AuNP(At)PEG or AuNP-PEG (non-labeled) to a subcutaneously transplanted kidney cancer model mouse.
Fig. 12 illustrates a mass of a tumor tissue extracted on day 40 after administration of AuNP(At)PEG or AuNP-PEG (non-labeled) to a subcutaneously transplanted kidney cancer model mouse.
Fig. 13 is a schematic diagram illustrating a structure of 5 nm PEG-AuNP(At)-c[RGDfK(C)] (5nm mPEG-S-AuNP[²¹¹At]-c[RGDfK(C)]).
Fig. 14 illustrates the results of scintigraphy in a subcutaneously transplanted glioma model rat. Illustrated are the results of scintigraphy 9 and 14 hours after administration in ligation of the left femoral artery after selective intraarterial administration of 5 nm PEG-AuNP(At)-c[RGDfK(C) to an artery dominating a tumor-transplanted region. The arrow indicates the location of a tumor subcutaneously implanted in the thigh.
Fig. 15 illustrates a change in body weight in a subcutaneously transplanted glioma model rat. Illustrated is a change in body weight of a rat after selective intraarterial administration of 5 nm PEG-AuNP(At)-c[RGDfK(C)]. IA-1 and IA-2 indicate data of rats subjected to administration of 5 nm PEG-AuNP(At)-c[RGDfK(C)], and Ctl-1 and Ctl-2 indicate data of control rats which are not subjected to administration of the drug, but only subjected to ligation of the left femoral artery.
Fig. 16 illustrates a change in tumor volume in a subcutaneously transplanted glioma model rat. Illustrated is a change in tumor volume after selective intraarterial administration of 5 nm PEG-AuNP(At)-c[RGDfK(C)]. IA-1 and IA-2 indicate data of rats subjected to administration of 5 nm PEG-AuNP(At)-c[RGDfK(C), and Ctl-1 and Ctl-2 indicate data of control rats which are not subjected to administration of the drug.
Fig. 17 illustrates the results of scintigraphy in an intraperitoneally transplanted glioma model mouse. Illustrated are the results of scintigraphy 9 and 14 hours after intraperitoneal administration of 5 nm PEG-AuNP(At)-c[RGDfK(C).
Fig. 18 illustrates a viability curve in an intraperitoneally transplanted glioma model mouse. Illustrated is a viability curve after intraperitoneal administration of 5 nm PEG-AuNP(At)-c[RGDfK(C)] 2 weeks after tumor transplantation (group A). "At" indicates mice (n=3) subjected to administration of 5 nm PEG-AuNP(At)-c[RGDfK(C)]. "Ctl" indicates mice (n=2) subjected to administration of physiological saline as a control.
Fig. 19 illustrates a change in body weight in an intraperitoneally transplanted glioma model mouse. Illustrated is a change in body weight after intraperitoneal administration of 5 nm PEG-AuNP(At)-c[RGDfK(C)] 1 week after tumor transplantation (group B). "At" indicates an average for mice (n=3) subjected to administration of 5 nm PEG-AuNP(At)-c[RGDfK(C)]. "Ctl" indicates an average for mice (n=3) subjected to administration of physiological saline as a control.

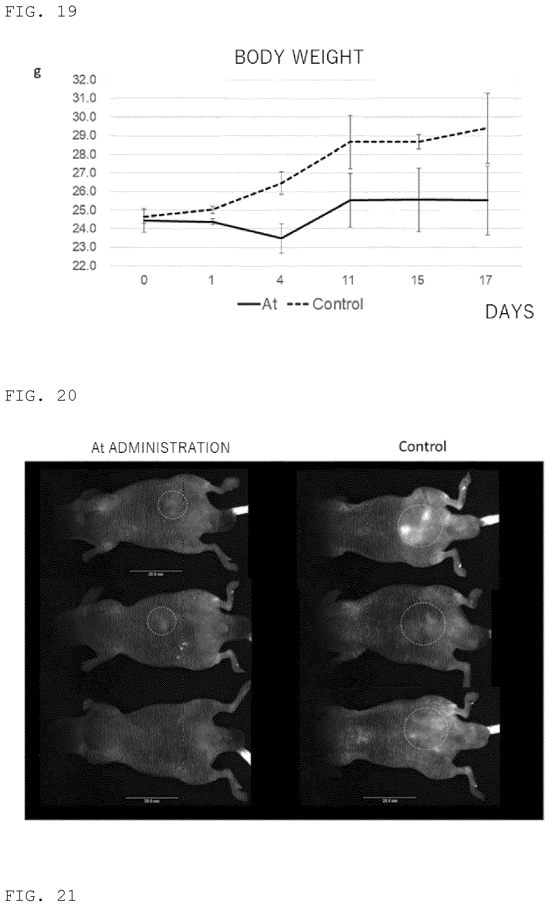

Fig. 20 is an image illustrating the fluorescent imager results of a tumor in an intraperitoneally transplanted glioma model mouse. Illustrated are the results of observation of a tumor state with a fluorescent imager after intraperitoneal administration of 5 nm PEG-AuNP(At)-c[RGDfK(C)] 1 week after tumor transplantation (group B). Illustrated are data obtained 3 weeks after tumor transplantation and 11 days after drug administration. "At administration" in the left column indicates the results for mice subjected to administration of 5 nm PEG-AuNP(At)-c[RGDfK(C)], and "Control" in the right column indicates the results for mice subjected to administration of physiological saline as a control.
Fig. 21 is an image illustrating the fluorescent imager results of a tumor in an intraperitoneally transplanted glioma model mouse. Illustrated are the results of observation of a tumor state with a fluorescent imager after intraperitoneal administration of 5 nm PEG-AuNP(At)-c[RGDfK(C)] 1 week after tumor transplantation (group B). Illustrated are data obtained 4 weeks after tumor transplantation and 18 days after drug administration. "At administration" in the left column indicates the results for mice subjected to administration of 5 nm PEG-AuNP(At)-c[RGDfK(C)], and "Control" in the right column indicates the results for mice subjected to administration of physiological saline as a control.

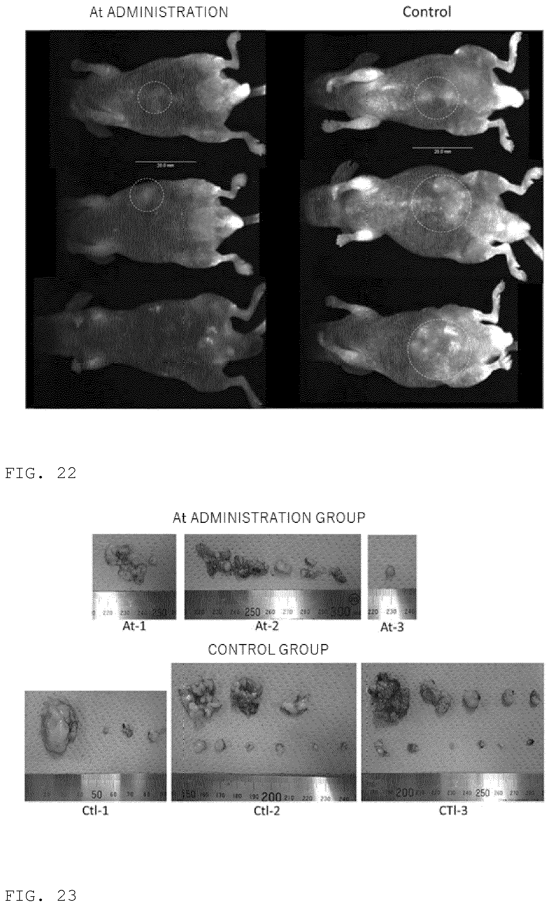

Fig. 22 is an image illustrating a tumor in an intraperitoneally transplanted glioma model mouse. Illustrated is an image of a tumor isolated 32 days after transplantation and 23 days after drug administration subsequent to intraperitoneal administration of 5 nm PEG-AuNP(At)-c[RGDfK(C)] 1 week after tumor transplantation into the peritoneal cavity (group B). "At administration group" (At-1, At-2 and At-3) indicates an image of a tumor isolated from a mouse subjected to administration of 5 nm PEG-AuNP(At)-c[RGDfK(C)], and "Control group" (Ctl-1, Ctl-2 and Ctl-3) indicates an image of a tumor isolated from a mouse subjected to administration of physiological saline as a control.
Fig. 23 is an image illustrating the results of fluorescent imaging of a tumor in an intraperitoneally transplanted glioma model mouse. Illustrated are the results of fluorescent imaging of a tumor isolated 32 days after transplantation and 23 days after drug administration subsequent to intraperitoneal administration of 5 nm PEG-AuNP(At)-c[RGDfK(C)] 1 week after tumor transplantation into the peritoneal cavity (group B). The "At administration group" (At-1, At-2 and At-3) indicates the results of a fluorescent imaging of a tumor isolated from a mouse subjected to administration of 5 nm PEG-AuNP(At)-c[RGDfK(C)], and the "Control group" (Ctl-1, Ctl-2 and Ctl-3) indicates the results of fluorescent imaging of a tumor isolated from a mouse subjected to administration of physiological saline as a control.

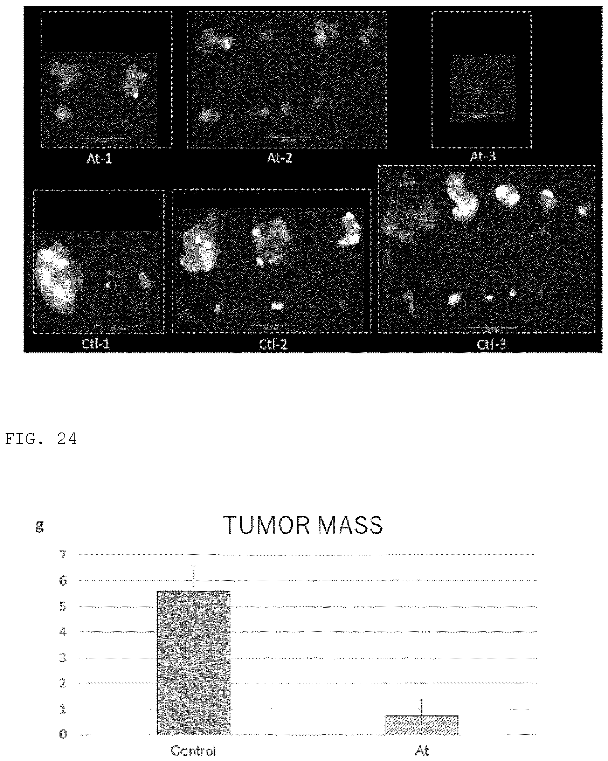

Fig. 24 illustrates a mass of a tumor in an intraperitoneally transplanted glioma model mouse. Illustrated is a mass of a tumor isolated 32 days after transplantation and 23 days after drug administration subsequent to intraperitoneal administration of 5 nm PEG-AuNP(At)-c[RGDfK(C)] 1 week after tumor transplantation into the peritoneal cavity (group B). "At" (indicates an average mass (n=3) of tumors isolated from mice subjected to administration of 5 nm PEG-AuNP(At)-c[RGDfK(C)], and "Control" indicates an average mass (n=3) of tumors isolated from mice subjected to administration of physiological saline as a control.

### Description of Embodiments

Hereinafter, embodiments of the present invention will be described. The following description is merely illustrative, and the scope of the present invention is not limited by the description, and can be appropriately changed and implemented as long as the spirit of the present invention is not impaired.

When At-211 is used as an alpha radioactive nucleus, At-211 has a property similar to that of iodine, and therefore rapidly accumulates in the thyroid gland when administered as it is. AuNPs can be used to control the drug distribution for retaining the drug in a tumor. Specifically, AuNP-PEG can be produced, purified, and then labeled with At-211 to produce At-211 AuNP(At)-PEG. The size of AuNPs can be appropriately adjusted depending on the purpose.

In an aspect, when At-211 AuNP(At)-PEG is administered into a malignant tumor, an appropriate amount of At-211 AuNP(At)-PEG (hereinafter referred to as nanoparticles) can be filled into a syringe, and slowly injected into the malignant tumor through an injection needle. Nanoparticles injected into the tumor diffuse throughout the tumor. The smaller the particle size, the better the degree of the diffusion, and it is known that the capillary blood vessel of a malignant tumor has higher substance permeability than that of a normal tissue, and when the particle size is small to a certain extent, the particles systemically diffuse through the capillary blood vessel.

On the other hand, when the particle size is about 100 nm, the diffusion in the tumor is deteriorated, so that it is not possible to obtain a sufficient effect. It has been found that when the particle size is about 30 nm or less, the particles diffusion well in the tumor, do not flow into a capillary blood vessel perfusing the tumor, and do not diffuse to other organs outside the tumor. The nanoparticles retained in the tumor continue irradiating the tumor cells with an alpha ray, and decay at a half-life of about 7 hours, and the radioactivity disappears. One injection damages the tumor cells by the alpha ray, and their ability to proliferate disappears or markedly decreases.

### (Definitions)

Herein, when a plurality of ranges of numerical values are indicated, a range consisting of a combination of any lower limit and any upper limit in each of the plurality of ranges has the same meaning.

Herein, the "alpha radioactive nucleus" means a nucleus that emits an alpha ray, and these alpha radioactive nuclei can be mixed, and used. As the nucleus that emits an alpha ray, At-211, Ac-225, Ra-223 and the like can be used, but the nucleus is not limited thereto. It is preferable to use At-211 as the alpha radioactive nucleus.

At-211 is a radioisotope of astatine (At) which is an element belonging to halogen. The At-211 disintegrates into stable lead (207 Pb) by emitting a high-energy alpha ray having a cell killing property. The half-life of At-211 is 7.2 hours. That is, At-211 has a short lifespan and a high cell killing property, and thus can efficiently destroy tumor tissue when used in tumor treatment.

These radiation-emitting nuclei can be manufactured by using a known method. For example, At-211 is manufactured by a (α,2n) nuclear reaction using an accelerator such as a cyclotron and using Bi-209 as a target substance, purified by a dry method (distillation), then dissolved in water, and supplied as an At-211 aqueous solution.

As used herein, the "proliferative disease" means a disease associated with undesired cell proliferation of one or more subsets of cells in a multicellular organism. Proliferative diseases can occur in various animals including humans. As used herein, the "proliferative disease" includes benign tumors, malignant tumors and other proliferative diseases. Examples of the "proliferative disease" include, but are not limited to, hematopoietic disorders (e.g. myeloproliferative disorders), malignant tumors (e.g. brain tumors, prostate cancer, head and neck cancer, oral cancer, breast cancer, pancreas cancer and digestive organ cancer).

As used herein, the "tumor " means a mass of tissue that is formed by autonomous and excessive proliferation against control in vivo. The "tumor" includes a "benign tumor" that is not considered malignant from a pathological point of view, and a "malignant tumor" that invades surrounding tissues or develops metastasis.

### (Method for synthesizing gold nanoparticles of invention)

The gold nanoparticles (AuNPs) of the present invention can be prepared by a known method. For example, the method described in J. Phys. Chem. C 2011, vol. 115, pp. 45024506 can be used without limitation. The size of the gold nanoparticles can be arbitrarily set by a method well known to those skilled in the art, and the size of the manufactured particles can be measured. For example, microscopy using a transmission electron microscope (TEM) can be used without limitation.

When the gold nanoparticles of the present invention are used as a malignant tumor treatment agent, the size of the gold nanoparticles can be appropriately selected depending on the type and state of a malignant tumor, a desired pharmacological effect, and the like. Preferably, the size of the gold nanoparticles is adjusted to the extent that the gold nanoparticles sufficiently diffuse in the tumor tissue, and do not flow into capillary blood vessels perfusing the tumor and diffuse to other organs outside the tumor on a widespread scale. In an aspect, the size of the gold nanoparticles is preferably 0.5 nm or more, 0.6 nm or more, 0.7 nm or more, 0.8 nm or more, 0.9 nm or more, 1 nm or more, 2 nm or more, 3 nm or more, 4 nm or more, 5 nm or more, 6 nm or more, 7 nm or more, 8 nm or more, 9 nm or more, 10 nm or more, 11 nm or more, 12 nm or more, 13 nm or more, 14 nm or more, 15 nm or more, 20 nm or more, 25 nm or more, or 30 nm or more. In an aspect, the size of the gold nanoparticles is 110 nm or less, 100 nm or less, 90 nm or less, 80 nm or less, 70 nm or less, 60 nm or less, 50 nm or less, 40 nm or less, or 30 nm or less. In an aspect, the size of the gold nanoparticles is preferably 1.0 nm to 110 nm. In another aspect, the size of the gold nanoparticles is preferably 5 nm to 30 nm.

The gold nanoparticles can be bound to the alpha radioactive nucleus by a known method. In particular, it is known that gold forms a stable bond with a halogen element (Dziawer L et al. RSC advances, 2017, Vol. 7, pp. 41024-41032), and when a halogen alpha radioactive nucleus such as At-211 is used as an alpha radioactive nucleus, the gold nanoparticles can be bound to the halogen alpha radioactive nucleus can by mixing the gold nanoparticles with the halogen alpha radioactive nucleus.

### (Modification of gold nanoparticles of invention)

By modifying the surfaces of the gold nanoparticles (AuNP) of the present invention, the behavior of the gold nanoparticles in vivo can be adjusted. For example, by modifying the surfaces of the gold nanoparticles (AuNPs) with polyethylene glycol, a saccharide, a peptide (protein), or another polymer, functions such as aggregation suppression, cell targeting, promotion of cell membrane permeation, and enhancement of cell membrane adsorption can be imparted to the gold nanoparticles in the tumor tissue. For the modification of the gold nanoparticles, hydrocarbon-based polymers such as polyethylene glycol, polyether and polyol, polyethyleneimine, silica gel, peptides, antibodies, proteins, lipids, complex lipids, sugar chains, complex carbohydrates, terpene, terpenoid, and virus-like particles can be used without limitation. Preferably, the gold nanoparticles of the present invention are modified with polyethylene glycol (PEG). As the PEG for use in the present invention, various polymers obtained by condensation polymerization of ethylene oxide and water and having various structures known to be used for biomaterials can be used, and PEG having no chemically reactive terminal group, monofunctional PEG having one chemically reactive terminal group, difunctional PEG having two chemically reactive terminal groups, linear PEG, multiarmed PEG, PEG having a reactive terminal group such as a N-hydroxysuccinimide ester group, a thiol group or a carboxy group, and the like can be used without limitation (Drug Delivery System, 2015, Vol. 30, No. 4, pp. 390-392). It is also possible to combine a plurality of modifications as long as their functions are not mutually inhibited.

The size of the polymer that is used for modifying the gold nanoparticles of the present invention can be appropriately set by those skilled in the art depending on the type and condition of a tumor to be treated, a desired effect, and the like. For example, when PEG is used, any polymer having a molecular size equal to or greater than that of diethylene glycol can be used. The molecular weight thereof is not particularly limited, and may be, for example, 100 or more, 200 or more, 300 or more, 400 or more, 500 or more, 600 or more, 700 or more, 800 or more, 900 or more, 1,000 or more, 1,500 or more, 2,000 or more, 2,500 or more 3,000, 11,000 or more, 3,500 or more, 4,000 or more, 4,500 or more, 5,000 or more, or 6,000 or more, and 7,000 or less, 8,000 or less, 12,000 or less, 13,000 or less, 14,000 or less, 15,000 or less, 16,000 or less, 17,000 or less, 18,000 or less, 9,000 19,000 or 10,000 less, or 20,000 or less. More specifically, PEG having a molecular weight of about 100 to 20,000, 200 to 19,000, 300 to 18,000, 400 to 17,000, 500 to 16,000, 1,000 to 15,000, 2,000 to 12,000, 3,000 to 10,000, 4,000 to 9,000, 5,000 to 8,000, 5,500 to 7,000, or 6,000 can be used. The molecular weight of PEG can be adjusted depending on modifications that are used in combination. Such modifications can be performed by a known method (e.g. Gold Bull. 2011, vol. 44, pp. 99-105).

### (Carrier of gold nanoparticles of invention)

When the gold nanoparticles of the present invention are administered to cells, various substances for regulating uptake into cells can be used in combination. For example, by encapsulating the gold nanoparticles in virus particles passing through a cell membrane, the gold nanoparticles can be efficiently delivered into cells. Modification with a compound that causes the surfaces of gold nanoparticles to have cationic charge, such as polyethyleneimine improves the cell permeability of the gold nanoparticles. Modification of the surface with an antibody or the like that causes cell-specific uptake improves the uptake of the gold nanoparticles into cells. As the targeting molecule that improves uptake into a specific cell, a molecule binding to a substance specifically in a proliferative disease cell, for example, an antibody having as an antigen a protein specifically expressed in a proliferative disease cell, or an antigen-binding fragment thereof; an antibody having, for example, CD19, EpCAM, CD20, CD45, EGFR, HER2 or CDH17 as an antigen, or an antigen-binding fragment thereof; a ligand binding to a receptor specifically expressed in a proliferative diseased cell, or a fragment thereof; substance P that is a ligand of a NK1 receptor expressed in, for example, glioma, or a fragment thereof, for example, a peptide consisting of 5 to 11 amino acids at the N-terminal, or another peptide having a tumor targeting function, for example, a cyclic peptide c[RGDfK(C)] (Vivitide (Kentucky, USA)) having a tumor targeting function; or the like can be used. The targeting molecule can be bound to the alpha radioactive nucleus-bound gold nanoparticles directly or with the surface-modifying molecule or another carrier interposed therebetween.

### (Pharmaceutical composition and treatment method)

The gold nanoparticles bound to an alpha radioactive nucleus according to the present invention are provided as a medicine for treating a proliferative disease or the like. The medicine containing the gold nanoparticles bound to an alpha radioactive nucleus according to the present invention is effective for treatment of various proliferative diseases, and can be applied to treatment of malignant tumors such as brain tumors, prostate cancer, head and neck cancer, oral cancer, breast cancer and digestive organ cancer. The gold nanoparticles bound to an alpha radioactive nucleus according to the present invention have excellent diffusion characteristics in a tumor tissue, and hardly transfer out of the tumor tissue. Therefore, even in treatment of malignant tumors with many blood vessels, such as brain tumors, it is possible to effectively treat malignant tumors while suppressing radiation exposure of other organs.

The present invention also provides a method for treating a proliferative disease or the like by using gold nanoparticles bound to an alpha radioactive nucleus.

The treatment of a malignant tumor includes suppression of progression, regression, elimination, suppression of metastasis and prevention of recurrence of a primary malignant tumor.

The gold nanoparticles bound to an alpha radioactive nucleus according to the present invention can be administered to humans or other mammals, for example, mice, rats, rabbits, sheep, pigs, cattle, cats, dogs and monkeys. The route of administration of a composition containing the gold nanoparticles of the present invention can be appropriately selected by those skilled in the art, and the composition is provided in a dosage form adapted to the route of administration. In an aspect, it is preferable that the gold nanoparticles bound to an alpha radioactive nucleus according to the present invention are topically administered to a lesion such as a tumor. Here, the gold nanoparticles can be administered by injection into the lesion with the use of an injection needle or the like (intratumoral administration). For example, an alpha radioactive nucleus-bound gold nanoparticle-containing liquid preparation having the same volume as the tumor volume can be injected into the central part of the tumor tissue over 1 minute while making an observation with an echo image.

As the topical administration, other methods obvious to those skilled in the art can be utilized in addition to injection into a tumor tissue as described above. For example, by using a catheter or the like, gold nanoparticles can be super-selectively administered into an artery feeding the lesion (super-selective intraarterial administration). In the super-selective intraarterial administration, it is possible to apply a method using a catheter, which has been remarkably developed in recent years. The administration method enables the drug to be administered to the disease tissue in every hole and corner and exclusively without directly invading the disease tissue. It is also possible to perform the administration by intracavitary application of the gold nanoparticles to a lesion seeded in a cavity such as a cavity left after removal of the tumor, the peritoneal cavity or the chest cavity (intracavitary administration). In the intracavitary administration, a high concentration of the drug can be administered to the seeded lesion. The administration method is useful as administration for prevention of recurrence in a cavity left after surgery.

By selecting the optimal particle size of the gold nanoparticles, these topically administered alpha radioactive nucleus-bound gold nanoparticles do not require use of a specific targeting molecule in the proliferative disease cells, and exhibit uniform distribution within the tissue once they reach the inside of the proliferative disease tissue, and flow of the gold nanoparticles into capillary blood vessels on the periphery of the outside of the tissue. Therefore, it is possible to suppress systemic radiation exposure while obtaining an effective proliferation inhibitory effect on proliferative disease cells.

The alpha radioactive nucleus-bound gold nanoparticles for use in the pharmaceutical composition or treatment method of the present invention may be modified or are not required to be modified by the surface modification as long as there is no contradiction to the purpose of the present invention. Preferably, the alpha radioactive nucleus-bound gold nanoparticles are subjected to surface modification with a hydrocarbon-based polymer such as polyethylene glycol (PEG). The alpha radioactive nucleus-bound gold nanoparticles for use in the pharmaceutical composition or the treatment method of the present invention enable systemic radiation exposure to be suppressed while obtaining an excellent proliferation inhibitory effect on proliferative disease cells without using a targeting molecule having affinity for a specific proliferative disease cell, for example, an antibody, another protein, a peptide or a low-molecular-weight compound. In an aspect, the alpha radioactive nucleus-bound gold nanoparticles for use in the pharmaceutical composition or treatment method of the present invention are subjected to surface modification with a hydrocarbon-based polymer which is not bound to a targeting molecule.

In an aspect, the alpha radioactive nucleus-bound gold nanoparticles for use in the pharmaceutical composition or treatment method of the present invention are subjected to surface modification with a hydrocarbon-based polymer which is not bound to a targeting molecule and the same hydrocarbon-based polymer or a different hydrocarbon-based polymer which is bound to a targeting molecule. The proportion of the hydrocarbon-based polymer which is not bound to a targeting molecule can be adjusted as long as there is no contradiction to the purpose of the present invention. The proportion of the hydrocarbon-based polymer which is not bound to a targeting molecule may be 10%, 20%, 30%, 40%, 50%, 60%, 70%, 80%, 90% of the total number of molecules of the hydrocarbon-based polymer used for the surface modification.

### (Method for selecting particle size of alpha radioactive nucleus-bound gold nanoparticles)

In an aspect, the present invention provides a method for selecting a particle size of alpha radioactive nucleus-bound gold nanoparticles which is optimum for treating a proliferative disease by topical administration. The method comprises the steps of: (1) providing alpha radioactive nucleus-bound gold nanoparticles having different particle sizes ranging from 0.5 to 110 nanometers; (2) administering the alpha radioactive nucleus-bound gold nanoparticles having respective particle sizes into a proliferative disease tissue in vivo; (3) confirming an alpha ray distribution in the proliferative disease tissue subjected to the administration, and a systemic alpha ray distribution; and (4) selecting a particle size on the basis of the alpha ray distribution in the proliferative disease tissue, and the systemic alpha ray distribution.

The method can be carried out by using a model animal in which a proliferative disease tissue is transplanted and proliferated, such as a model animal having a proliferative disease tissue or a cancer-bearing model animal. The alpha ray distribution in the proliferative disease tissue and the systemic alpha ray distribution can be evaluated by using methods known to those skilled in the art. For example, the evaluation can be performed by image analysis using scintigraphic analysis, autoradiographic analysis or the like. According to the evaluation, it is preferable to select a gold nanoparticle size which exhibits a systemic alpha ray distribution exhibiting a low-level alpha ray distribution outside the proliferative disease tissue while exhibiting a relatively uniform alpha ray distribution in the proliferative disease tissue.

Those skilled in the art can determine the specific uniformity of the specific alpha ray distribution in the proliferative disease tissue and level of the alpha ray distribution outside the proliferative disease tissue by using a statistical index according to a desired effect. For the uniformity of the alpha ray distribution in the proliferative disease tissue, for example, a radioactivity distribution obtained by scintigraphy or autoradiography can be visually evaluated. Alternatively, texture analysis is performed, a value of entropy or the like is used as an index, and it is evaluated that the uniformity is higher when the value is low. In these evaluations, the uniformity is preferably higher. The level of the alpha ray distribution outside the proliferative disease tissue can be evaluated by, for example, measuring radioactivity in each organ by scintigraphy or SPECT. The radioactivity measurement value in each organ can be measured from an image. It is preferable that the measured value is close to zero. When radioactivity is measured, an exposure dose is calculated by using dedicated software such as OLINDA/EXM, and the exposure dose is preferably low.

On the basis of the results of the measurement and evaluation, a particle size of the gold nanoparticles which gives higher uniformity of the alpha ray distribution in the proliferative disease tissue and a lower level of the alpha ray distribution outside the proliferative disease tissue is selected.

The method of the present invention may comprise (5) evaluating a change in body weight of an animal subjected to the administration, and/or an inhibitory effect on proliferation of the proliferative diseased tissue, in addition to the step (4), and selecting a particle size on the basis of these evaluations. The inhibitory effect on proliferation of the proliferative disease tissue can be determined by, for example, comparison with a control in terms of a change in volume of the proliferative disease tissue after the administration of the alpha ray-bound gold nanoparticles and/or the mass of the proliferative disease tissue after the elapse of a certain period after the administration.

The topical administration into the proliferative disease tissue can be selected from the group consisting of injection to the central part of the tissue, super-selective administration into an artery feeding the lesion, and application of the drug into a cavity where the tissue is present. The in vivo proliferative disease tissue may be a heterogeneous-proliferative disease tissue transplanted into a subject.

The surface of the gold nanoparticle may be modified with a molecule selected from hydrocarbon-based polymers such as polyethylene glycol, polyether and polyol, polyethyleneimine, silica gel, a peptide, an antibody, a protein, a lipid, a complex lipid, a sugar chain, a complex carbohydrate, terpene, terpenoid, and a virus-like particle. In an aspect, the surface may be modified with polyethylene glycol having a molecular weight of 2,000 to 20,000.

The proliferative disease is a malignant tumor, and may be a solid cancer. The solid cancer may be selected from a brain tumor, endocrine tumors, prostate cancer, head and neck cancer, oral cancer, breast cancer, gynaecological cancer, skin cancer, pancreas cancer, and digestive organ cancer.

The alpha radioactive nucleus-bound gold nanoparticles having a particle size selected by the method of the present invention exhibit uniform distribution within the tissue once they reach the inside of the proliferative disease tissue, and flow of the gold nanoparticles into capillary blood vessels on the periphery of the outside of the tissue. Therefore, the method of the present invention enables selection of the particle size of gold nanoparticles having excellent characteristics as an active ingredient of a medicine which ensures that systemic radiation exposure is suppressed while a sufficient inhibitory effect on proliferation of proliferative disease cells is obtained.

### (Method for selecting particle size of alpha radioactive nucleus-bound gold nanoparticles and manufacturing the gold nanoparticles)

In an aspect, the present invention provides a method for manufacturing alpha radioactive nucleus-bound gold nanoparticles having a particle size optimum for treating a proliferative disease by topical administration. The method comprises the step of selecting a particle size on the basis of the method for selecting a particle size of the alpha radioactive nucleus-bound gold nanoparticles, and manufacturing alpha radioactive nucleus-bound gold nanoparticles by using golf nanoparticles having the selected particle size.

The alpha radioactive nucleus-bound gold nanoparticles manufactured by the method of the present invention exhibit uniform distribution within the tissue once they reach the inside of the proliferative disease tissue, and flow of the gold nanoparticles into capillary blood vessels on the periphery of the outside of the tissue. Therefore, the method of the present invention enables manufacturing of gold nanoparticles having excellent characteristics as an active ingredient of a medicine which ensures that systemic radiation exposure is suppressed while a sufficient inhibitory effect on proliferation of proliferative disease cells is obtained.

Hereinafter, the present invention will be described in more detail by way of Examples, which should not be construed as limiting the scope of the present invention. Examples

### (1) Synthesis of AuNP(At)PEG

Hydrogen tetrachloroaurate(III) tetrahydrate (Kishida Chemical Co., Ltd. (Osaka, Japan)) was used as a raw material for synthesis of gold nanoparticle. For labeling gold nanoparticles with PEG, poly(ethylene glycol) methyl ether thiol (Mₙ 6,000) (Sigma-Aldrich Co, LLC (St. Louis, USA)) was used.

The quality of the synthesized PEG-labeled gold nanoparticles was confirmed by transmission electron microscope (TEM) (JEM-2100, JEOL Ltd. (Tokyo, Japan)) imaging. The radioactivity of AuNP(At)PEG was measured with a germanium semiconductor detector (BE-2020, Mirion Technologies (Canberra), Inc. (Connecticut, USA)).

The following aqueous solutions A to E were used for the synthesis of AuNP.

Aqueous solution A was adjusted by adding 8 mL of water to 2 mL of a hydrogen tetrachloroaurate(III) tetrahydrate (0.17% w/v) aqueous solution.

Aqueous solution B was adjusted by mixing 0.5 mL of an ascorbic acid (1% w/v) aqueous solution and 0.25 mL of a trisodium citrate (0.88% w/v) aqueous solution, and adding 9.25 mL of water.

Aqueous solution C was adjusted by adding 2 mL of water to 8 mL of a hydrogen tetrachloroaurate(III) tetrahydrate (0.17% w/v) aqueous solution.

Aqueous solution D was adjusted by mixing 2 mL of an ascorbic acid (1% w/v) aqueous solution and 1 mL of a trisodium citrate (0.88% w/v) aqueous solution, and adding 7 mL of water.

AuNP(At)PEG was administered to rats and mice after being appropriately diluted with physiological saline.

### (1-1) Preparation of 5 nm AuNP

5 nm AuNP was purchased from Sigma-Aldrich Co, LLC (St. Louis, USA)).

### (1-2) Synthesis of 13 nm AuNP

47.5 mL of water was added to 2.5 mL of a hydrogen tetrachloroaurate(III) tetrahydrate (0.17% w/v) aqueous solution. After completion of the addition, the temperature was raised to 100 degrees or higher with stirring. After the temperature was raised, 2 mL of an aqueous solution obtained by mixing trisodium citrate (0.88% w/v) and citric acid (0.05% w/v) was added, and the mixture was stirred at the same temperature for 5 minutes. After the stirring, the temperature was brought back to room temperature to obtain 13 nm AuNP. By TEM imaging, it was confirmed that the average particle size of AuNPs was 13 nm (13.1 ± 1.4 nm).

### (1-3) Synthesis of 30 nm AuNP

15 mL of water was added to 5 mL of the 13 nm AuNP aqueous solution to perform adjustment. While the adjusted aqueous solution was stirred, aqueous solutions A and B were simultaneously added from separate syringes at a flow rate of 0.25 mL/min. After completion of the addition, the temperature was raised to 100 degrees or higher with stirring, and the mixture was stirred at the same temperature for 30 minutes. After the stirring, the temperature was brought back to room temperature to obtain 30 nm AuNP. By TEM imaging, it was confirmed that the average particle size of AuNPs was 30 nm (30.8 ± 2.7 nm).

### (1-4) Synthesis of 60 nm AuNP

15 mL of water was added to 5 mL of the 30 nm AuNP aqueous solution to perform adjustment. While the adjusted aqueous solution was stirred, aqueous solutions A and B were simultaneously added from separate syringes at a flow rate of 0.25 mL/min. After completion of the addition, the temperature was raised to 100 degrees or higher with stirring, and the mixture was stirred at the same temperature for 30 minutes. After the stirring, the temperature was brought back to room temperature to obtain 60 nm AuNP. By TEM imaging, it was confirmed that the average particle size of AuNPs was 60 nm.

### (1-5) Synthesis of 120 nm AuNP

While 20 mL of the 60 nm AuNP aqueous solution was stirred, aqueous solutions C and D were simultaneously added from separate syringes at a flow rate of 0.25 mL/min. After completion of the addition, the temperature was raised to 100 degrees or higher with stirring, and the mixture was stirred at the same temperature for 30 minutes. After the stirring, the temperature was brought back to room temperature to obtain 120 nm AuNP. By TEM imaging, it was confirmed that the average particle size of AuNPs was 120 nm (120.7 ± 13.3 nm).

### (1-6) Modification of 5nm, 13nm, 30nm and 120nm AuNP with PEG

To each of 5 nm, 13 nm, 30 nm and 120 nm AuNP aqueous solutions, poly(ethylene glycol) methyl ether thiol (Mₙ 6,000) was added to a final concentration of 0.1 mg/mL. Thereafter, the mixture was stirred at room temperature for 2 hours. After stirring, ultrafiltration (10,000 G, 10 min) was performed on the 5 nm AuNP-PEG aqueous solution, and distilled water was added. This procedure was carried out a total of three times to obtain a 5 nm AuNP-PEG aqueous solution freed of impurities. For the 13 nm AuNP-PEG aqueous solution, the 30 nm AuNP-PEG aqueous solution and the 120 nm AuNP-PEG aqueous solution, AuNP-PEG was precipitated by centrifugation (10,000 G, 1 hr). Thereafter, the supernatant was removed by decantation, and distilled water was added in an amount equal to that of the removed solution. This procedure was carried out a total of two times to obtain a 13 nm AuNP-PEG aqueous solution, a 30 nm AuNP-PEG aqueous solution and a 120 nm AuNP-PEG aqueous solution freed of impurities. By TEM imaging, it was confirmed that the AuNP surface was modified with PEG.

### (1-7) Labeling of AuNP-PEG with At-211

### (Labeling of 5 nm AuNP-PEG with At-211)

An At-211 aqueous solution was added to the 5 nm AuNP-PEG aqueous solution, and the mixture was shaken at room temperature for 15 minutes. After the shaking, a 5 nm AuNP(At)PEG (5 nm mPEG-S-AuNP[²¹¹At]) aqueous solution (about 42.3 MBq/mL) was obtained.

### (Labeling of 13 nm AuNP-PEG with At-211)

An At-211 aqueous solution was added to the 13 nm AuNP-PEG aqueous solution, and the mixture was shaken at room temperature for 15 minutes. After the shaking, a 13 nm AuNP(At)PEG (13 nm mPEG-S-AuNP[²¹¹At]) aqueous solution (about 40.7 MBq/mL) was obtained.

### (Labeling of 30 nm AuNP-PEG with At-211)

An At-211 aqueous solution was added to the 30 nm AuNP-PEG aqueous solution, and the mixture was shaken at room temperature for 15 minutes. After the shaking, a 30 nm AuNP(At)PEG (30 nm mPEG-S-AuNP[²¹¹At]) aqueous solution (about 39.0 MBq/mL) was obtained.

### (Labeling of 120 nm AuNP-PEG with At-211)

An At-211 aqueous solution was added to the 120 nm AuNP-PEG aqueous solution, and the mixture was shaken at room temperature for 15 minutes. After the shaking, a 120 nm AuNP(At)PEG (120 nm mPEG-S-AuNP[²¹¹At]) aqueous solution (about 39.9 MBq/mL) was obtained.

The mass concentration of the AuNP(At)-PEG aqueous solution of each size was measured by using ICP-OES (Optima 8300, Perkin Elmer Inc. (Waltham, USA)), and the particle concentration was calculated. By using these values, the particle concentration in the AuNP(At)-PEG aqueous solution to be administered to in vitro and in vivo experimental models was adjusted, so that the doses of radiation to be administered were approximately the same. In the in vitro experiment, the concentration was adjusted with distilled water, and in the in vivo experiment, the concentration was adjusted with physiological saline.

In the in vitro experiment, the solution was continuously diluted and administered as described below, and in the in vivo experiment, the solution was administered with preparation performed so that the dose per animal was the radiation dose shown in Table 1 below.

**[Table 1]**

| Model | Nanoparticle | Diameter (nm) | Particle concentration (/mL) | Mass concentration (mg/L) | Dose (MBq) |
|---|---|---|---|---|---|
| C6 | 5 nm AuNP(At)- PEG | 5.1 | 9.46 × 10¹² | 12.7 | 1.39 |
| | | (1.1 nm) | (1.65 × 10¹²) | (2.21) | (0.04) |
| | 13 nm AuNP(At)- PEG | 13.1 | 2.46 × 10¹¹ | 5.59 | 1.46 |
| | | (1.4) | (4.92 × 10¹⁰) | (1.12) | (0.07) |
| | 30 nm AuNP(At)-PEG | 30.8 | 2.95× 10¹⁰ | 8.74 | 1.29 |
| | | (2.7) | (2.76 × 10¹⁰ ) | (8.18) | (0.50) |
| | 120 nm AuNP(At)- PEG | 120.7 | 2.38 × 10⁹ | 42.4 | 1.51 |
| | | (13.3) | (2.53 × 10⁹) | (45.0) | (0.57) |
| | 30 nm AuNP-PEG | 30.8 | 6.49 × 10¹⁰ | 19.2 | 0 |
| | | (2.7) | | | |
| PANC-1 | 13 nmAuNP(At)- PEG | 13.1 | 1.15 × 10¹⁵ | 26.2 | 1.17 |
| | | (1.4) | (1.94 × 10¹⁴) | (4.40) | (0.07) |
| | 13 nm AuNP- PEG | 13.1 | 1.23 × 10¹⁵ | 27.8 | 0 |
| | | (1.4) | (2.13 × 10¹⁴) | (4.83) | |

| | | | | | |
|---|---|---|---|---|---|
| "Mass concentration" indicates a mass concentration of Au in the solution. *The numbers in brackets are standard deviations. | | | | | |

### (1-8) Synthesis of 5 nm PEG-AuNP(At)-c[RGDfK(C)]

An aqueous solution of poly(ethylene glycol) methyl ether thiol (Mₙ 350) (Biochempeg Scientific Inc. (Massachusetts, USA)) and c [RGDfK(C)] (Vivitide (Kentucky, USA)) was added to the 5 nm AuNP aqueous solution. The final concentrations of poly (ethylene glycol) methyl ether thiol (Mₙ 350) and c[RGDfK(C)] were each adjusted to 0.1 mM. Thereafter, the mixture was stirred at room temperature for 2 hours. After stirring, ultrafiltration (10,000 G, 10 min) was performed on the 5 nm PEG-AuNP-c[RGDfK(C)] aqueous solution, and distilled water was added. This procedure was carried out a total of three times to obtain a 5 nm PEG-AuNP-c[RGDfK(C)] aqueous solution freed of impurities. An At-211 aqueous solution was added to the 5 nm PEG-AuNP-c[RGDfK(C)] aqueous solution, and the mixture was shaken at room temperature for 15 minutes. After the shaking, 5nm PEG-AuNP(At)-c[RGDfK(C)] (5nm mPEG-S-AuNP[²¹¹At]-c[RGDfK(C)]) (about 58.5 MBq/mL) was obtained.

### (2)In vitro cytotoxicity test

### (2-1) Tumor cell

C6 glioma and PANC-1 cells (TACC (Virginia, USA)) were used. These cells were cultured in a humidified culture vessel under the conditions of 37°C and added 5% CO2 by using DMEM medium (FUJIFILM Wako Pure Chemical Corporation (Osaka, Japan)) containing 10% fetal bovine serum (GibcoTM, Life Technologies, Carlsbad, (CA USA)) and a 1% penicillin-streptomycin solution (FUJIFILM Wako Pure Chemical Corporation (Osaka, Japan)).

### (2-2) Test method

C6 glioma cells (2 × 10⁴ cells/well in 100 µL medium) and PANC-1 cells (1 × 10⁴ cells/well in 100 µL medium) were seeded in a 96-well plate and cultured for 1 day. AuNP-PEG (without a radiation nucleus) and AuNP(At)-PEG having different particle sizes were synthesized, and a test solution was prepared in such a manner that the radiation doses per mL of AuNP(At)-PEGs having the various particle sizes were approximately the same. The adjusted test solution was continuously diluted and added to each well to a radiation dose of 0 to 1 MBq/mL (25 µL/well). After culturing for 24 hours, the cell viability was measured with a 450 nm microplate reader by using Cell Counting Kit 8 (CCK8) (DOJINDO LABORATORIES. (Kumamoto, Japan)).

### (2-3) Results

When C6 glioma cells treated with 5 nm, 13 nm, 30 nm or 120 nm AuNP-PEG without addition of a radioactive nucleus were cultured for 24 hours, the cell viability was not influenced even in the case where the cells were treated at a high concentration. That is, AuNP-PEG without addition of a radiation nucleus had no toxicity regardless of the particle size and the concentration. On the other hand, in C6 glioma and PANC-1 cells subjected to administration of 120 nm AuNP(At)-PEG with At-211, which has a particle size of 120 nm, at a radiation dose of 1 MBq/mL, there was a marked decrease in viability.

C6 glioma was cultured with AuNP-PEG for 24 hours, and as a result, 5 nm, 13 nm, 30 nm and 120 nm AuNP(At)-PEGs were internalized in C6 glioma cells, but 5 nm, 13 nm and 30 nm AuNP(At)-PEGs were internalized only at a high concentration in contrast to 120 nm AuNP(At)-PEG. On the other hand, only 120 nm AuNP(At)-PEG was precipitated in the solution, and it was considered that in 120 nm AuNP(At)-PEG, the cell-peripheral local concentration of AuNP-PEG increased in the well. From the above results, it thought that AuNP-PEG and AuNP(At)-PEG were incorporated into cells in a concentration-dependent manner. In an experiment conducted on PANC-1 cells instead of C6 glioma cells, similar results were obtained, suggesting that the cytotoxicity of AuNP(At)-PEG did not depend on the cell type.

### (3) Animal model

### (3-1) Rat

Male nude rats (F344/NJcl-rnu/rnu (CLEA Japan, Inc. (Tokyo, Japan))) (7 weeks old) were used.

### (3-2) Mouse

Male nude mice (BALB/C Slc-nu/nu (Japan SLC, Inc. (Tokyo, Japan))) (5 weeks old) were used.

### (3-3) Tumor cells

As glioma cells, C6 glioma cells, a cell line of rat glioma obtained by introduction of N-nitrosomethylurea, were used (obtained from RIKEN BRC). C6 glioma cells were cultured in a humidified culture vessel under the conditions of 37°C and added 5% CO2 by using MEM medium (Sigma-Aldrich Japan, (Tokyo, Japan)) containing 10% fetal bovine serum.

As pancreas cancer cells, human pancreas cancer cells PANC-1 were used (obtained from American Type Culture Collection). PANC-1 was cultured in RPMI 1640 medium containing L-glutamine and phenol red (FUJIFILM Wako Pure Chemical Corporation (Tokyo, Japan)), 10% heat-inactivated fetal bovine serum and 1% penicillin-streptomycin.

### (3-4) Transplantation of tumor cells into rat

50 µl matrigel (Corning (New York, USA)) was added to 0.9 × 10^7 C6 glioma cells/50 µl MEM, and the cells were subcutaneously transplanted to both sides of the back of a rat under anesthesia using 2.0% isoflurane in oxygen.

50 µl matrigel (Corning (New York, USA)) was added to 1.0 × 10^7 human pancreas cancer PANC-1 cells/50 µl RPMI 1640, and the cells were subcutaneously transplanted into the left shoulder of a mouse.

### (3-5) Synthesis of AuNP

13 days after the transplantation of C6 glioma cells, the rat (n=11) was subjected to intratumoral drug administration under anesthesia with the use of 2.0% isoflurane in oxygen. Physiological saline (n=3), AuNP(At)PEG having a diameter of 120 nm + physiological saline (n=4), and AuNP(At)PEG having a diameter of 30 nm + physiological saline (n=4) were each provided in an amount equal to the tumor volume, and slowly administered into the tumor on each of both sides over about 1 minute. For the administration, a linear probe of an ultrasonic apparatus (ProSound α6, Hitachi-Aloka Medical, Ltd. (Tokyo, Japan)) was used, and a needle tip of a syringe (Myjector 29 G, Terumo Co. Ltd. (Tokyo, Japan)) was placed at the center of the tumor. For the radioactive agent, the dose of radioactivity per tumor during administration was 1.4 ± 0.5 MBq. After the administration, an alpha survey meter (TCS-232B, Hitachi Co. Ltd. (Tokyo, Japan)) was used to confirm that there was not contamination resulting from backflow of the radioactive drug into the skin.

In the test where 5 nm, 13 nm, 30 nm or 120 nm AuNP(At)PEG was administered to the rat, the rat (n=12) was subjected to intratumoral drug administration under anesthesia with the use of 2.0% isoflurane in oxygen 13 days after the transplantation of C6 glioma cells. Physiological saline (3 rats, 6 tumors), 30 nm AuNP-PEG without addition of a radiation nucleus + physiological saline (3 rats, 6 tumors), 120 nm AuNP(At)-PEG + physiological saline (4 rats, 8 tumors), 30 nm AuNP(At)-PEG + physiological saline (4 rats, 8 tumors), 13 nm AuNP(At)-PEG + physiological saline (3 rats, 6 tumors), and 5 nm AuNP(At)-PEG + physiological saline (3 rats, 6 tumors) were each provided in an amount equal to the tumor volume. For control animals, similarly, physiological saline (3 rats, 6 tumors) and 30 nm AuNP-PEG without addition of a radiation nucleus + physiological saline (3 rats, 6 tumors) were provided. These test solutions were each slowly administered over about 1 minute. For the administration, a linear probe of an ultrasonic apparatus (ProSound α6, Hitachi-Aloka Medical, Ltd. (Tokyo, Japan)) was used, and a needle tip of a syringe (Myjector 29 G, Terumo Co. Ltd. (Tokyo, Japan)) was placed at the center of the tumor. For the radioactive agent, the dose of radioactivity per tumor during administration was 1.4 ± 0.4 MBq (Table 1). After the administration, an alpha survey meter (TCS-232B, Hitachi Co. Ltd. (Tokyo, Japan)) was used to confirm that there was not contamination resulting from backflow of the radioactive drug into the skin.

### (3-6) Autoradiography

All the tumors of rats (n=1) subjected to administration of AuNP(At)PEG having an average diameter of 120 nm and rats (n=1) subjected to AuNP(At)PEG having a diameter of 30 nm were removed and rapidly frozen at -80°C the day after the administration. After isolation of the tumors, the rats were euthanized by administration of an excessive amount of isoflurane. The tumor was sliced to a thickness of about 30 um with a cryostat (CryoStar NX70, Thermo Scientific Inc. (MA, USA)), and attached to slide glass. Thereafter, the frozen section was quickly dried with a dryer, and then made to keep contacting with the imaging plate for about 1 hour. The imaging was performed by using a nonconfocal variable mode laser scanner (Typhoon FLA 7000, GE Healthcare Life Sciences (Buckinghamshire, England)).

### (3-7) Scintigraphic analysis

For rats subjected to administration of AuNP(At)PEG, scintigraphy was performed 4 hours and 19 to 21 hours after the administration. Scintigraphy was performed with a low-energy, high-resolution and a parallel-hole collimator mounded on a gamma camera (E.cam, Siemens Healthcare (Erlangen, Germany)). The rat was fixed prone on a bed under anesthesia using 2.0% isoflurane in oxygen, and imaging was performed for 10 minutes after 4 hours and for 30 minutes after 19 hours to obtain planar images of the front and back surfaces with a 128 × 128 matrix size.

### (3-8) Statistics

Statistical calculation was performed by using SPSS 17.0. For comparison of tumor masses, one-way ANOVA and Levene's test were conducted, followed by conduction of a post-test using Tukey's HSD test.

All experiments were conducted in accordance with Osaka University Regulations on Animal Experiments after reception of the consent of the Osaka University Animal Experiment Committee. The results of the experiments are reported in accordance with ARRIVE (Animal Research: Reporting in Vivo Experiments) guideline.

### (4)Injection of AuNP(At)PEG into subcutaneously transplanted glioma

### (4-1) Scintigraphic analysis

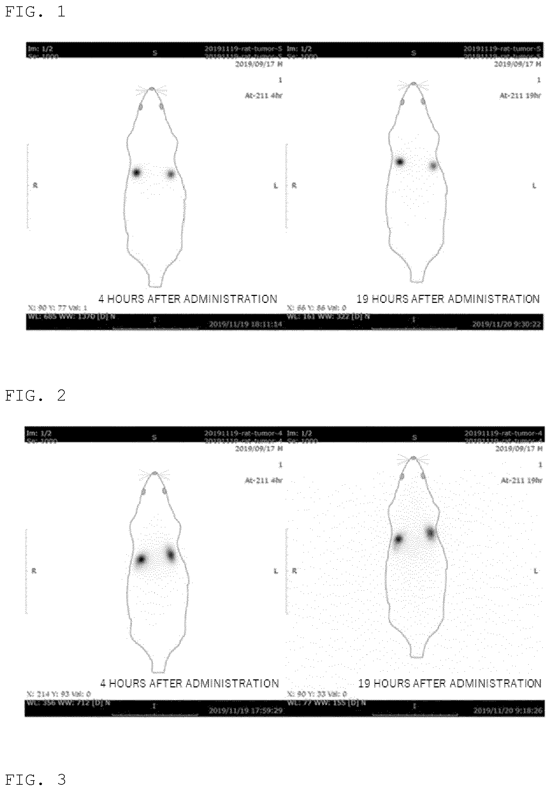

Physiological saline alone, 120 nm AuNP(At)PEG + physiological saline, or 30 nm AuNP(At)PEG + physiological saline was injected into the tumors on both sides of C6 glioma cell-implanted rats. Scintigraphic analysis was performed at 4 and 19 hours after the administration. Results for 120 nm AuNP(At)PEG (Fig. 1) and 30 nm AuNP(At)PEG (Fig. 2) are shown.

A signal was not detected at sites other than the tumor in either 120 nm AuNP(At)PEG or 30 nm AuNP(At)PEG.

On the other hand, 120 nm AuNP(At)PEG accumulated in dots in the tumor tissue (Fig. 1), whereas 30 nm AuNP(At)PEG diffused in the tumor tissue (Fig. 2).

As a reference experiment, AuNP(I-123)PEG obtained by binding I-123 as a gamma ray nucleus to the same AuNP-PEG was administered. The results are shown in Fig. 3. The binding between I-123 and AuNP is unstable, and 1.5 hours after the injection into the subcutaneous tumor (arrow) on the left side, I-123 is relatively rapidly separated from AuNP, flows into the blood vessel, and is distributed throughout the body (Fig. 3).

### (4-2) Autoradiography

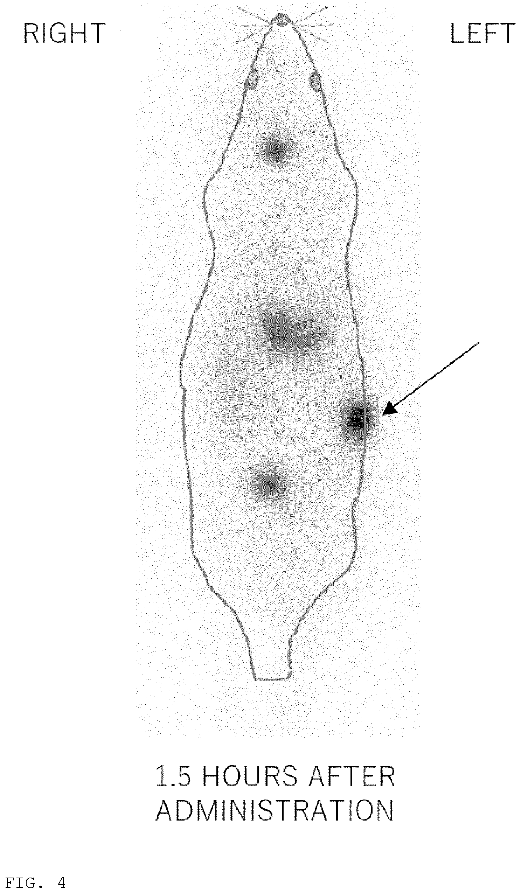

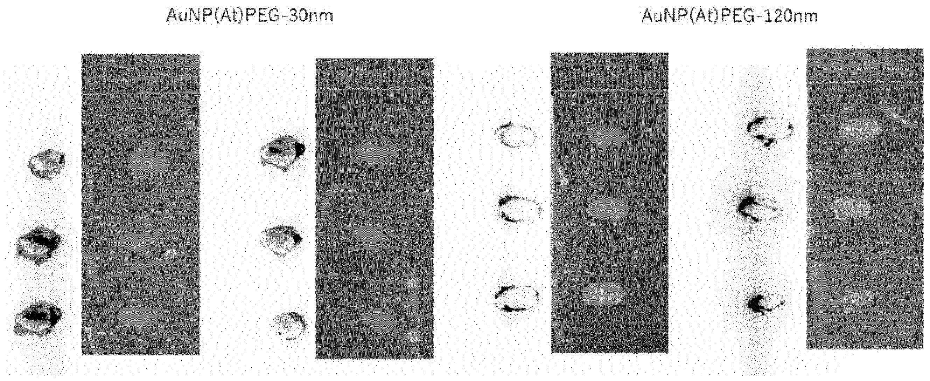

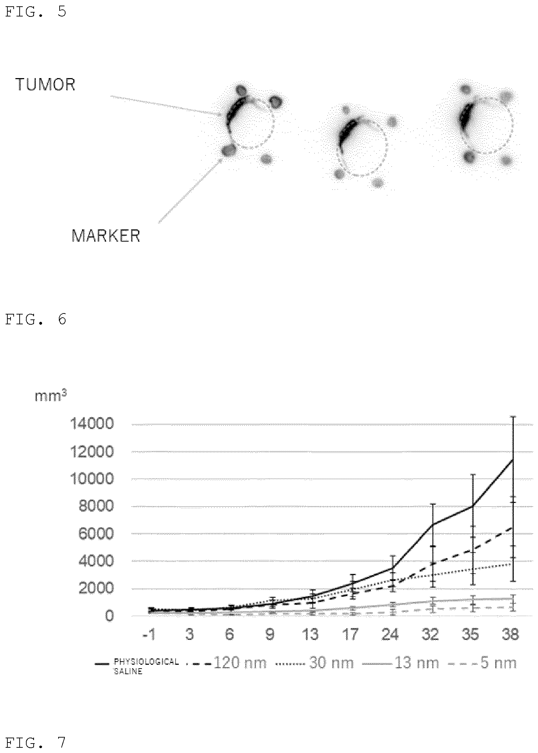

From the rat injected with 120 nm AuNP(At)PEG or 30 nm AuNP(At)PEG, the tumor tissue was extracted the day after the injection, and subjected to autoradiography. The results are shown in a figure (Fig. 4). It was confirmed that 120 nm AuNP(At)PEG was localized in the marginal part of the tumor (Fig. 4B), whereas 30 nm AuNP(At)PEG was distributed throughout the tumor (Fig. 4A). In 30 nm AuNP(At) which is not modified with PEG, the distribution in the tumor tended to be slightly biased (Fig. 5).

### (4-3) Inhibitory effect on proliferation of tumor

The antitumor actions of 5 nm, 13 nm, 30 nm and 120 nm AuNP(At)PEGs were compared by using a rat subcutaneously implanted C6 glioma model.

Saline alone, or each of 5 nm, 13 nm, 30 nm and 120 nm AuNP(At)PEGs was injected into the tumor together with physiological saline, follow-up observation of the rat was then performed for 38 or 39 days, and during the follow-up, the body weight and the tumor size were measured under anesthesia using 2.0% isoflurane in oxygen. 40 days after the administration, the tumor was isolated, and the mass of the tumor was measured. After the experiment, the animals were euthanized by administration of an excessive amount of isoflurane.

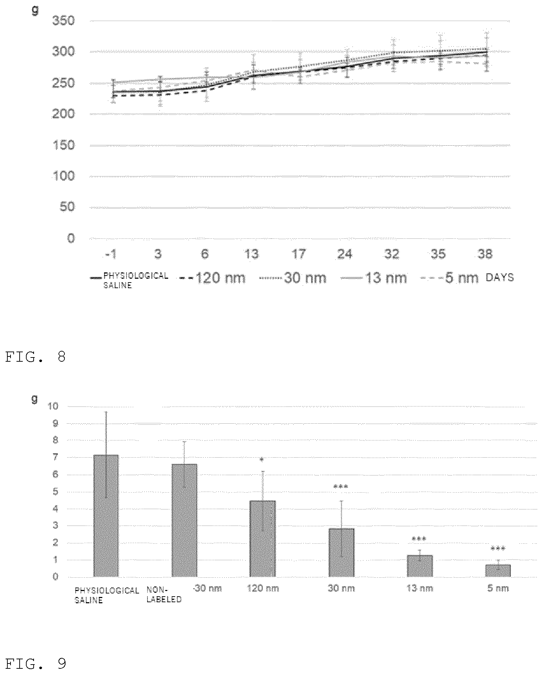

Fig. 6 shows a change in tumor size, Fig. 7 shows a change in body weight, and Fig. 8 shows comparison of the masses of extracted tumors.

Comparison of the tumor sizes themselves in rats subjected to administration of AuNP(At)PEGs having the various particle sizes showed that for these AuNP(At)PEGs, tumors of rats subjected to administration of AuNP(At)PEG having a smaller particle size had a smaller increase in tumor size, and the increase in tumor size was the smallest in rats subjected to administration of 5 nm AuNP(At)PEF (Fig. 6). There was no marked difference in change in body weight (Fig. 7). The masses of tumor tissues extracted from the rats 40 days after the administration were measured, and the results showed that tumors of rats subjected to administration of AuNP(At)PEG having a smaller particle size had a smaller mass, and the tumor mass was smallest in rats subjected to administration of 5 nm AuNP(At)PEF (Fig. 8). For rats subjected to administration of 30 nm AuNP-PEG without addition of At-211, the tumor mass was substantially the same as that in rats subjected to administration of physiological saline. From these results, it was confirmed that AuNP(At)PEG particles exhibited an excellent antitumor effect when their particle size was as small as 5 nm.

For the 5 nm AuNP(At)PEG particles, scintigraphic analysis was performed 4 hours, 19 hours and 42 hours after injection of 5 nm AuNP(At)PEG particles were injected into subcutaneous tumors on both sides as in (3-1) above, and the results shown in Fig. 9 were obtained. From these results, it was confirmed that AuNP(At)PEG particles having a small particle size of 5 nm were retained in the tumor tissue, and diffusion thereof outside the tumor tissue was limited.

Without being bound by any theory, it is believed that AuNP(At)PEG particles having a small particle size of 5 nm provide an excellent antitumor effect because they have excellent diffusion characteristics in a tumor tissue while their diffusion from the tumor tissue is limited. From the results of these experiments, it was confirmed that without use of a targeting molecule specific to proliferative disease cells, adjustment of the particle size enabled manufacturing of alpha ray gold nanoparticles having extremely excellent characteristics as a radioactive treatment drug such that the nanoparticles are uniformly distributed in the tumor tissue and their diffusion outside the tumor tissue is limited.

### (4-4) Tumor proliferation inhibitory effect on pancreas tissue

Human pancreas cancer PANC-1 cells were transplanted into the left shoulder of each of 12 nude mice (BALB/cSlc-nu/nu) (male, 5 weeks old). 14 days after the transplantation, 13 nm AuNP(At)mPEG was administered to 6 mice, and unlabeled 13 nm AuNPmPEG was administered to 6 mice as a control. The administration method was the same as that for the rats. The tumor size and the mouse body weight were measured for 39 days after administration. 40 days after the transplantation, the tumor was isolated, and the mice were euthanized.

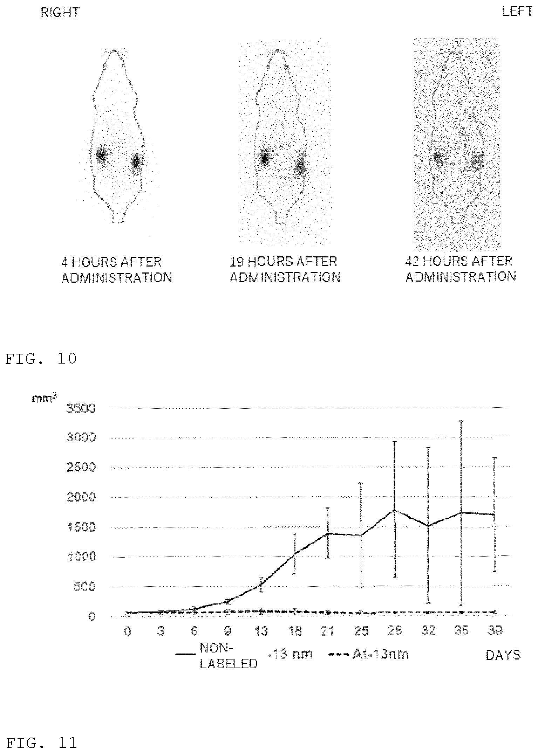

From scintigraphy, it was confirmed that the radioactive nuclei were not distributed in areas other than the tumor until 42 hours after the administration. In mice subjected to administration of AuNP(At)mPEG, the proliferative ability of the tumor markedly decreased as compared to the control (Fig. 10). On the other hand, the body weight of each of the control mice significantly decreased as compared to the group subjected to administration of AuNP(At)mPEG (p = 0.006) (Fig. 11).

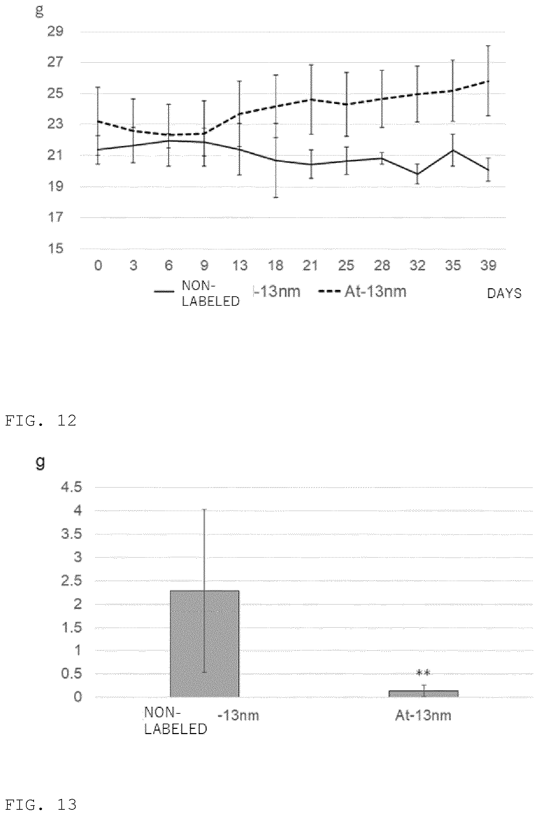

Comparison of the masses of isolated tumors showed that the tumor mass was significantly smaller in the group subjected to administration of AuNP(At)mPEG than in the control (Fig. 12). (p=0.006). From these experiments, it was confirmed that topical administration of AuNP(At)mPEG according to the present invention is also effective in treatment of pancreas cancer.

### (5) Selective intraarterial administration to subcutaneous transplantation model

As a preliminary experiment on C6 for clarifying the vascular control of the artery, a catheter was inserted under direct view into a branch of the left femoral artery of each of four 8-week-old nude rats (F344/NJcl-rnu/rnu(CLEA Japan, Inc. (Tokyo, Japan)), an appropriate amount of indocyanine green was injected to visually evaluate staining of the tissue, thereby confirming that the subcutaneous inner part of the distal part of the left thigh was the control region of the artery. C6 cells were transplanted to the subcutaneous inner side of the left thigh where staining was observed.

14 days after the transplantation, the groin was incised under isoflurane anesthesia, and a catheter was placed under direct vision into the branch of the femoral artery so as to be directed to the periphery. Thereafter, a drug with 0.3 ml of Dormicum (midazolam 1.5 mg), 0.3 ml of Seractal (xylazine 6 mg) and 0.4 ml of Vetorphale (butorphanol tartrate 0.8 mg) was intramuscularly administered at 0.07 ml/100 g, and the rat was anesthetized. Thereafter, 0.7 ml of Adenoscan (adenosine: 2 mg) was bolus-administered to two rats through the tail vein for the purpose of temporarily lowering the pulse and blood pressure to reduce the flow rate of blood, and immediately thereafter, 3.3 MBq of 5 nm PEG-AuNP(At)-c[RGDfK(C)] was dissolved in 0.3 ml of physiological saline, and administered slowly (over about 1.5 minutes) from the catheter placed in the femoral artery. The structure of 5nm PEG-AuNP(At)-c[RGDfK(C)] (5nm mPEG-S-AuNP[²¹¹At]-c[RGDfK(C)]) is shown in Fig. 13.

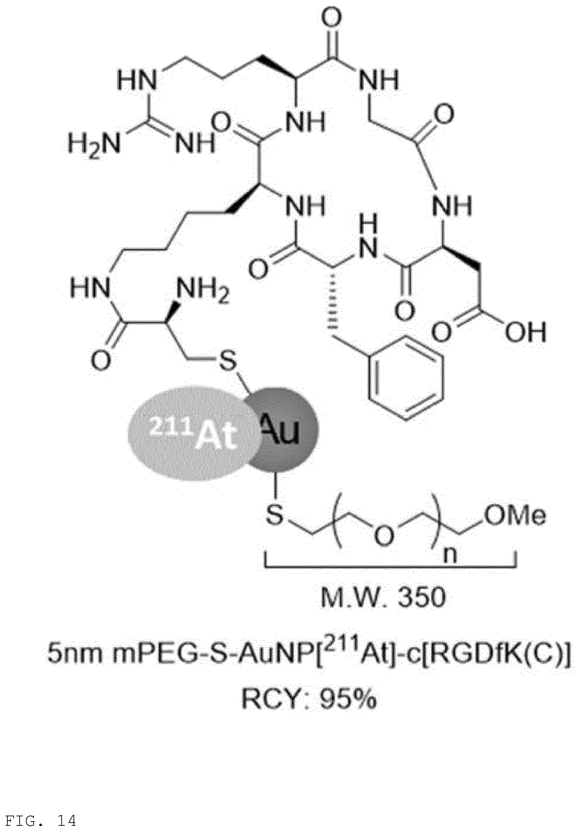

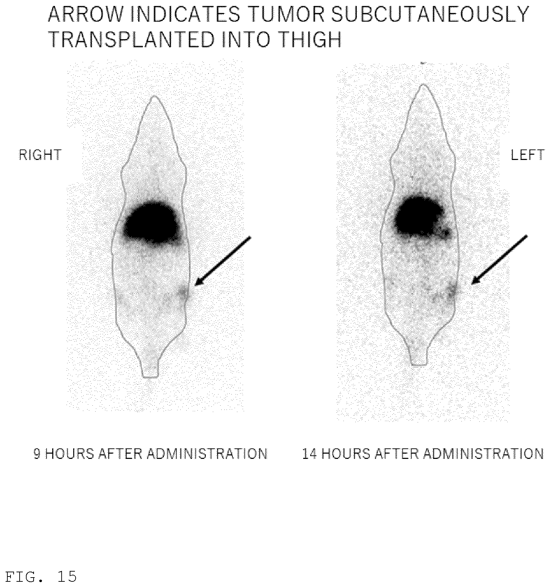

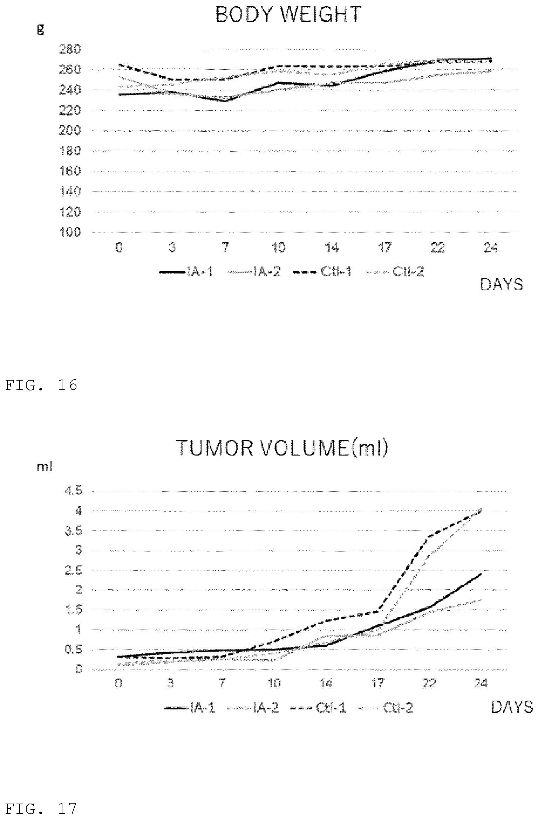

After the administration, the catheter was removed and the femoral artery was ligated. For two rats in the control group, a surgery was carried out in which the femoral artery was ligated, and the surgical incision was closed. For rats subjected to administration of At, the systemic distribution of the drug was evaluated by scintigraphy 9 hours and 14 hours after the administration. At any of the time points, RI accumulated in the tumor, the liver and the spleen, and did not apparently accumulate in other organs (Fig. 14). Thereafter, a change in body weight and the tumor volume were observed (Figs. 15 and 16), and the results showed that there was no apparent body weight difference between the rat subjected to administration of At and the control (Fig. 15). On the other hand, from the results of observation of the tumor volume, it was confirmed that the tumor proliferation was suppressed as compared to the rat subjected to administration of At and the control (Fig. 16). These experiments revealed that selective intraarterial administration of 5 nm PEG-AuNP (At)-c[RGDfK(C)] suppressed tumor proliferation. These results indicate that gold nanoparticles having a specific particle size and bound to an alpha radioactive nucleus according to the present invention exhibit an excellent treatment effect for a proliferative disease by super-selective administration into an artery feeding a lesion.

### (6) Experiments of intraperitoneal administration to peritoneal seeding model

1 × 10⁷ C6 cells containing a fluorescent protein gene were intraperitoneally transplanted to six 8-week-old male nude mice (group A) and six 9-week-old male mice (group B) (BALB/C Slc-nu/nu (Japan SLC, Inc. (Tokyo, Japan))). 5 nm PEG-AuNP(At)-c[RGDfK(C)] was intraperitoneally administered at 0.98 ± 0.19 MBq/0.2 ml to each of three mice with a 26G needle 14 days after the transplantation in group A and 7 days after the transplantation in group B. To each of three control animals in each of both the groups, 0.2 ml of physiological saline was administered with a 26 G needle.

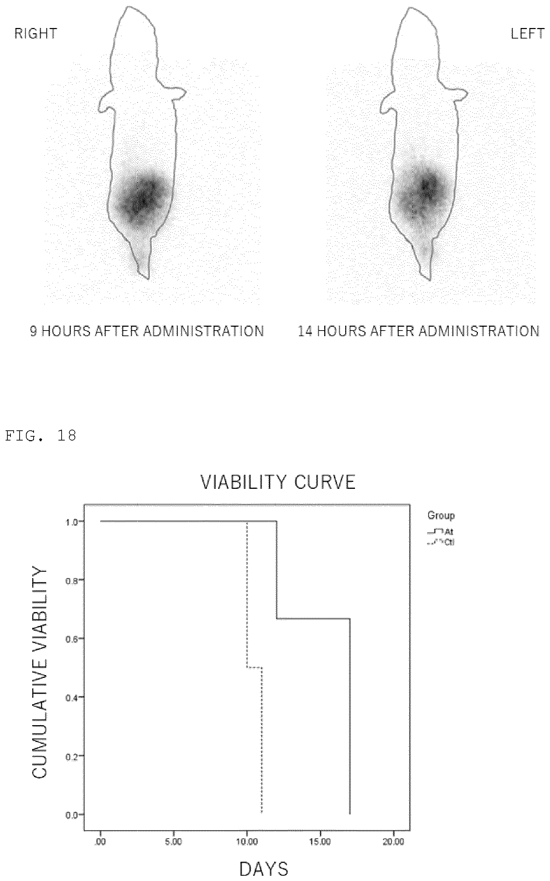

The systemic distribution of the drug was evaluated by scintigraphy 9 hours and 14 hours after the administration (Fig. 17). At any of the time points, RI was localized in the peritoneal cavity, and was not distributed in other organs. Tumor engraftment was not observed in one control animal of group A, and therefore this animal was excluded. Tumor engraftment was observed in all other animals. The average time of death in group A was 10.5 days after the administration for the control and 15.3 days after the administration for the At administration group (Fig. 18). In group B, all animals lived until 32 days after the transplantation (23 days after the administration). For group B, observation of a change in body weight and observation of the peritoneal cavity with a fluorescent imager were performed, and the results showed that in the control animals, there was an increase in body weigh probably due to an increased ascites as compared to the drug administration animals (Fig. 19).

4 weeks after the transplantation (17 days after the administration), a tumorous lump in the abdominal cavity was tangible in the control animals, and fluorescence was observed in the relevant part by observation with a fluorescence imager. On the other hand, in the At administration animals, the tumor was not tangible, or a nodule was tangible, and slight fluorescence was observed in the relevant part. 32 days after transplantation (23 days after administration), tumors were isolated, and the masses of the isolated tumors were compared. The results showed that in the control animals, the tumor was more widespread and had a larger mass as compared to the At administration animals (Figs. 20 to 24). Fluorescence, which was not uniform, was observed in the isolated tumor (Fig. 23). These experiments revealed that intraperitoneal administration of 5 nm PEG-AuNP (At)-c[RGDfK(C)] enabled suppression of progression of a C6 peritoneal seeding lesion. The results indicate that gold nanoparticles having a specific particle size and bound to an alpha radioactive nucleus according to the present invention exhibit an excellent treatment effect for a proliferative disease by intracavity application.

The above results of experiments indicate that by administering the gold nanoparticles having a specific particle size and bound to an alpha radioactive nucleus according to the present invention, various proliferative diseases can be treated safely and effectively. It has been confirmed that use of gold nanoparticles having a particle diameter below the relevant range may cause flow out of the tumor tissue.

## Claims

1. A medicine for treating a proliferative disease, comprising gold nanoparticles having a particle size of 0.5 to 110 nanometers and bound to At-211, wherein the medicine is topically administered.

2. The medicine according to claim 1, wherein the surface of the gold nanoparticle may be modified with a molecule selected from polyethylene glycol, polyether, polyol, polyethyleneimine, silica gel, a peptide, an antibody, a protein, a lipid, a complex lipid, a sugar chain, a complex carbohydrate, terpene, terpenoid, and a virus-like particle.

3. The medicine according to claim 2, wherein the surface modification comprises a molecule that is not bound to a targeting molecule for a specific cell.

4. The medicine according to claim 3, wherein in the surface modification, the molecule that is not bound to a targeting molecule for a specific cell is polyethylene glycol having an average molecular weight of 2,000 to 20,000.

5. The medicine according to any one of claims 1 to 4, wherein the particle size of the gold nanoparticle is 0.5 to 13 nanometers.

6. The medicine according to any one of claims 1 to 5, wherein the topical administration is selected from the group consisting of injection into a lesion, super-selective administration into an artery feeding the lesion, and intracavity application.

7. The medicine according to any one of claims 1 to 6, wherein the proliferative disease is selected from a brain tumor, endocrine tumors, prostate cancer, head and neck cancer, oral cancer, breast cancer, gynaecological cancer, skin cancer, pancreas cancer, and digestive organ cancer.

8. A gold nanoparticle which has a particle size of 0.5 to 110 nanometers, binds to At-211 and includes a surface modification with polyethylene glycol that is not bound to a targeting molecule for a specific cell.

9. A method for manufacturing At-211-bound gold nanoparticles having an optimum particle size for treating a proliferative disease by topical administration, the method comprising the steps of:
(1) providing At-211-bound gold nanoparticles having different particle sizes ranging from 0.5 to 110 nanometers;
(2) administering the At-211-bound gold nanoparticles having respective particle sizes into a proliferative disease tissue in vivo;
(3) confirming an alpha ray distribution in the proliferative disease tissue subjected to the administration, and a systemic alpha ray distribution; and
(4) selecting a particle size on the basis of the alpha ray distribution in the proliferative disease tissue, and the systemic alpha ray distribution.
